# EUROPEAN PATENT SPECIFICATION

(11) **EP 1 568 305 B1**
(45) Date of publication and mention of the grant of the patent: **02.07.2014**
(21) Application number: 05004145.8
(22) Date of filing: 25.02.2005
(51) Int. Cl.: A61B 1/005, A61B 1/018

(54) **Endoscope treatment system**
Endoskopisches Behandlungssystem
Système de traitement endoscopique

(30) Priority: 26.02.2004 JP 2004051364
(43) Date of publication of application: 31.08.2005
(73) Proprietor: Olympus Corporation, Shibuya-ku, Tokyo (JP)
(72) Inventor: Okada, Tsutomu, Tachikawa-shi Tokyo (JP)
(74) Representative: von Hellfeld, Axel

(56) References cited:
- EP-A- 1 188 517
- DE-A1- 10 325 086
- US-A- 6 059 719
- PATENT ABSTRACTS OF JAPAN vol. 1999, no. 01, 29 January 1999 (1999-01-29) & JP 10 262900 A (OLYMPUS OPTICAL CO LTD), 6 October 1998 (1998-10-06)
- PATENT ABSTRACTS OF JAPAN vol. 018, no. 285 (C-1206), 31 May 1994 (1994-05-31) & JP 06 054801 A (OLYMPUS OPTICAL CO LTD), 1 March 1994 (1994-03-01)
- PATENT ABSTRACTS OF JAPAN vol. 2000, no. 04, 31 August 2000 (2000-08-31) & JP 2000 000207 A (OLYMPUS OPTICAL CO LTD), 7 January 2000 (2000-01-07)

## Description

### BACKGROUND OF THE INVENTION

### 1. Field of the Invention

The present invention relates to an endoscope treatment system.

### 2. Description of the Related Art

In the related art, an instrument used with an endoscope is operated by an assistant positioned near an operator of the endoscope, by being inserted through a forceps channel of the endoscope. However, there is a problem of the treatment not proceeding smoothly if the operator and the assistance do not communicate well. Another drawback arises where the operator cannot hold an insertion portion of the endoscope when the operator operates the instrument manually with his hand which does not hold the endoscope.

Therefore, several technologies were proposed which seek to perform the automatic operation of the instrument by the operator of the endoscope. Thus, JP-A-2003-111769 describes, in Fig. 1, a technology for providing an instrument driving unit separately from an endoscope body and the instrument, engaging an operating element of the instrument with the instrument driving unit, and then activating with a foot switch. In JP-A-6-54801, Fig. 1 describes a technology of operating by attaching and detaching the, treatment unit automatically to/from an arm portion integrated in the system. In JP-A-2000-207, in Figs. 6 and 8, a technology for connecting a power supply device and a foot switch as members provided separately from the endoscope to an electrical power source unit disposed detachably in the endoscope is disclosed.

However, according to the technology disclosed in JP-A-2003-111769, there are problems that the scale of the structure of the instrument driving unit increases, and hence a large space of installation is required. According to the technology disclosed in JP-A-6-54801, there is the problem that the scale of the system of the endoscope is increased and the structure becomes complicated. According to the technology disclosed in JP-A-2000-207, since the electrical power source unit and the foot switch for supplying electric power to an opening-closing unit are disposed apart from the endoscope, wiring for connecting them to the opening-closing unit is exposed outside and hence has to be run on the floor, whereby the area around the feet becomes cluttered.

In view of the foregoing, it is a general object of the present invention to provide a compact endoscope treatment system which enables an operator of the endoscope to operate a treatment portion with his/her hand operating the endoscope in a simple structure without creating clutter in the area around the feet.

JP 10262900 relates to an endoscopic system, wherein an endoscope consists of an insertion part having a leading curved end part and an operation part, and an operation knob is provided in the operation part to manipulate the leading end curved part. Processing equipment installed inside a channel of the leading end of the insertion part for the insertion of the processing equipment consists of an insertion part formed from a flexible tube and a gripper to grip living body tissue and the like. The processing equipment is opened/closed by advancing/retreating the end part of the gripper. The open/close operation is performed by turning on electricity to a piezoelectric actuator and the leading end the processing equipment is curved by winding up an operation wire by a motor. The driving of the piezoelectric actuator and the motor is controlled by a drive controller connected to an open/close switch and a curve-switch of an operation panel.

US 6,059,719 refers to an endoscope system comprising a plurality of endoscope modules having different treatment instruments mounted therein, an endoscope to which the endoscope modules can be attached, and a coupling member fixed to the distal part of an insertion unit of the endoscope and used to make the plurality of endoscope modules freely exchangeable. The plurality of endoscope modules having the different treatment instruments mounted therein are freely exchangeable to be coupled to the distal part of the insertion unit of the endoscope.

JP 06054801 relates to an endoscope with multifunction treating implement, wherein in the tip of an inserting part, a treatment unit is provided, and its treatment unit consists of a freely curvable arm part, and plural and effectors attached to this arm part so as to be freely attachable, detachable and changeable. Also, this endoscope is provided with a driving means for operating the treatment unit, a mechanism for controlling an operation of the driving means in accordance with an input of an input means for instructing an operation executed by this driving means, and connecting and separating the arm part and the end effector, a carrying passage for a change treating implement for guiding a transfer to the end effector, and a transfer driving means for collecting and supply between a treating implement changing mechanism for containing plural treating implements, and also, supplying the selected treating implement to the carrying passage and the treatment unit.

JP06054801 discloses an endoscope system according to the pre-amble of claim 1.

### BRIEF SUMMARY OF THE INVENTION

The present invention is an endoscope treatment system having the features of independent claim 1.

The instruction member which gives driving instructions to the driving-power source can cause the driving-power source to drive the back-and-forth mechanism to, in turn, cause the transmitting member to move back-and-forth in the axial direction with respect to the sheath member to transmit the drive force to the treatment portion. Therefore, when the operator operates the instruction member, the treatment portion provided on the instrument body can be driven by a driving-power.

The instrument body is provided with a driving member connected to the transmitting member to move back-and-forth responsive to the back-and-forth drive force from the back-and-forth mechanism, and a base member is included as well. In this arrangement, since the driving member can be moved back-and-forth with respect to the base member, the transmitting member can be moved back-and-forth in the axial direction with respect to the sheath member by transmitting the back-and-forth drive force of the back-and-forth mechanism to the transmitting member via the driving member.

The back-and-forth mechanism may be structured to be disposed in the endoscope body so that the structure of the instrument body is simplified. Alternatively, it may be disposed in the instrument body so that the structure of the endoscope body is simplified.

In any case, since the sheath and the transmitting member are driven with respect to each other, the back-and-forth mechanism preferably includes a first engaging member for engaging with the transmitting member and a second engaging member for engaging with the sheath member.

The driving-power source is a motor, which provides a rotational drive source. When using the rotational drive source, a converting mechanism is used for converting the rotational movement of the driving-power source to the back-and-forth. Such a converting mechanism may include a mechanism wherein the transmitting member is wound around the outer periphery of a rotatable roller or any suitable mechanism.

The back-and-forth mechanism is preferably located at a position opposed to an operating element of the endoscope with the intermediary of a forceps port.

Thus, the endoscope treatment system of the invention provides a compact system housing in a simple structure, in which the operator can operate the treatment portion with the same hand as that used for operating the endoscope, allowing the other hand to be used freely, for example, for holding the insertion portion.

### BRIEF DESCRIPTION OF THE SEVERAL VIEWS OF THE DRAWINGS

These and other features, aspects, and advantages of the apparatus and methods of the present invention will become better understood with regard to the following description, appended claims, and accompanying drawings where:

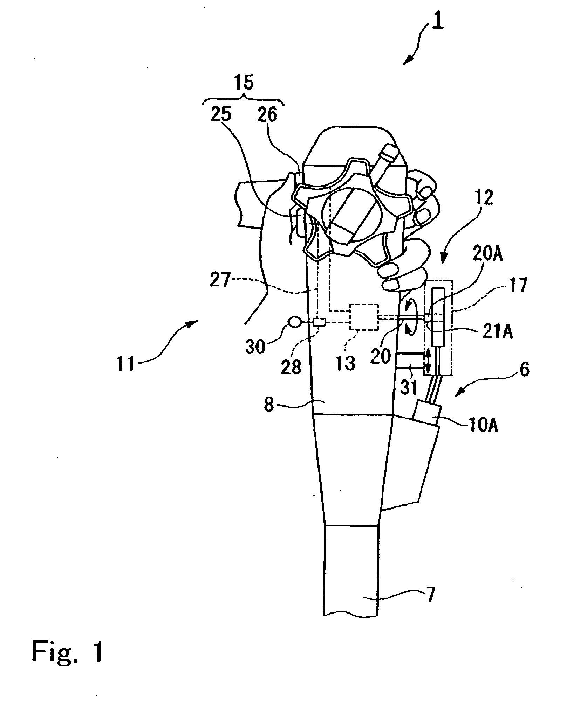

Fig. 1 is a side view, partly in cross-section, showing an endoscope treatment system according to a first embodiment of the present invention;

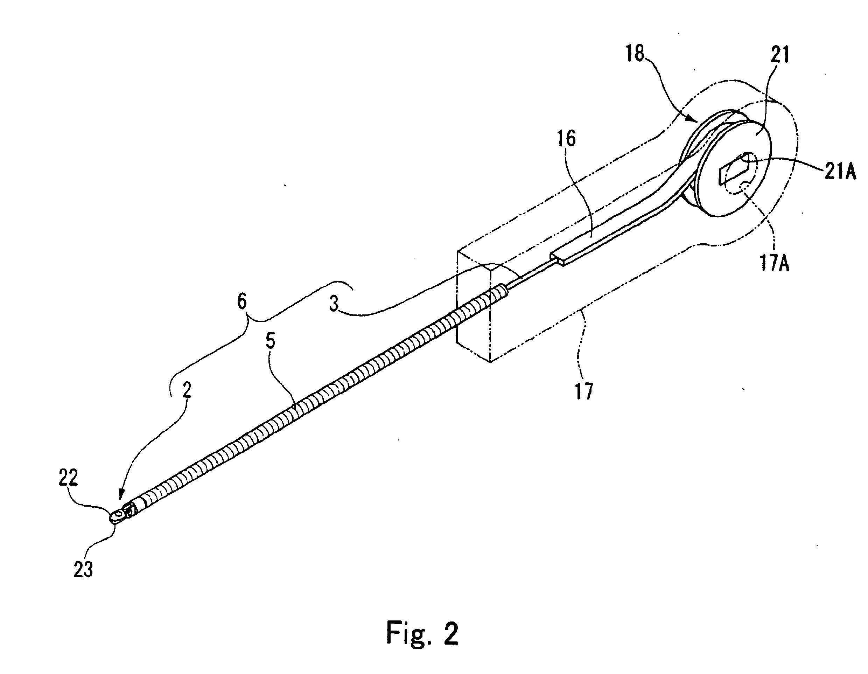

Fig. 2 is a perspective view showing an instrument body of the endoscope treatment system according to the first embodiment of the invention;

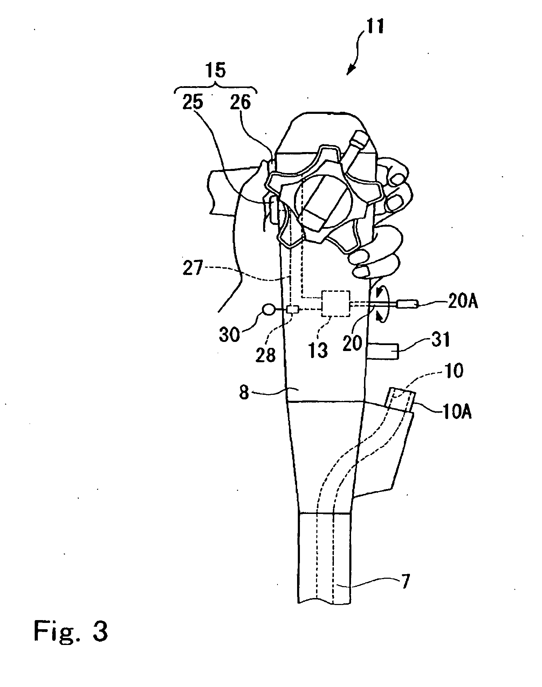

Fig. 3 is a side view showing an endoscope body of the endoscope treatment system according to the first embodiment of the invention;

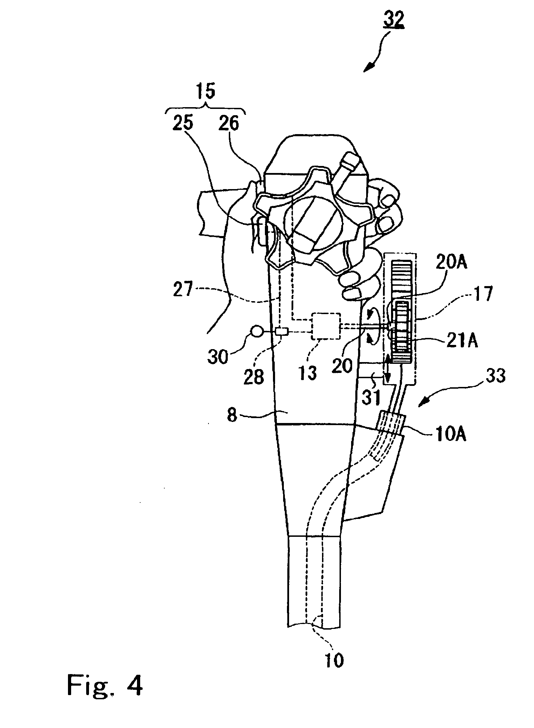

Fig. 4 is a side view partly in cross-section showing the endoscope treatment system according to a second exemplary embodiment useful for understanding the invention;

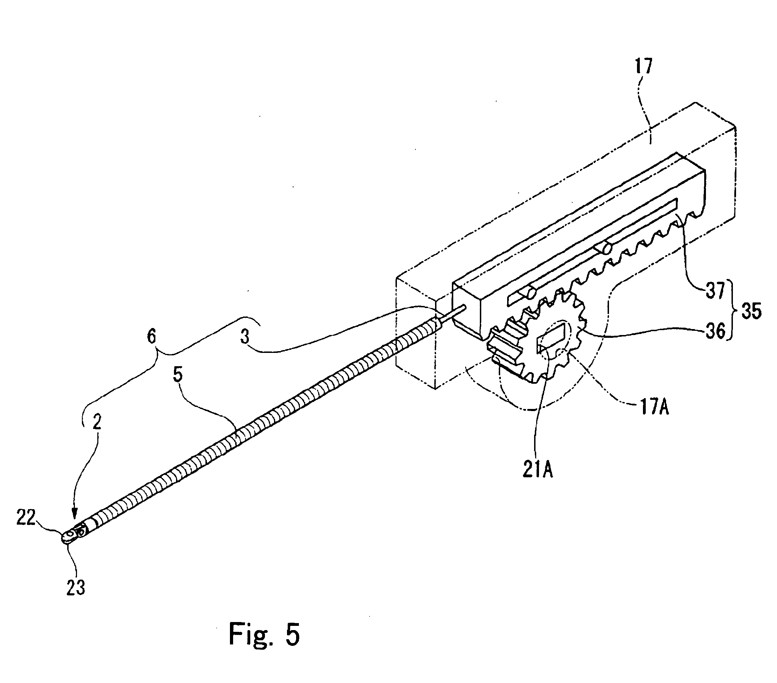

Fig. 5 is a perspective view showing the instrument body in the endoscope treatment system according to the second exemplary embodiment useful for understanding the invention;

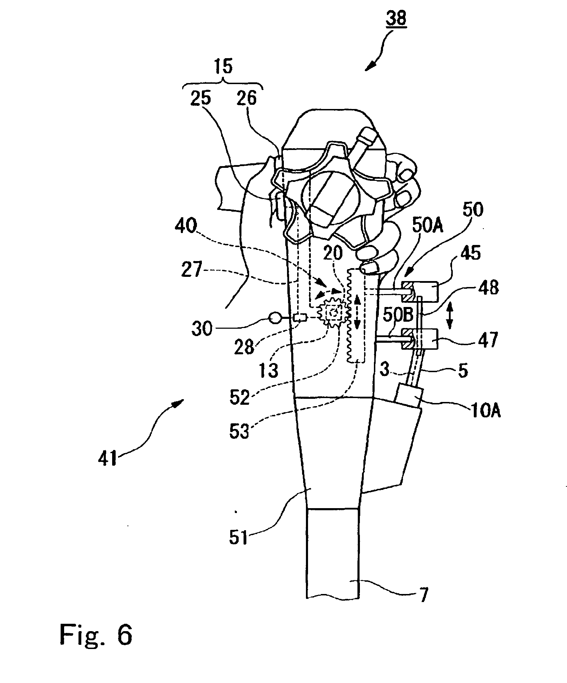

Fig. 6 is a side view partly in cross-section showing the endoscope treatment system according to a third exemplary embodiment useful for understanding the invention;

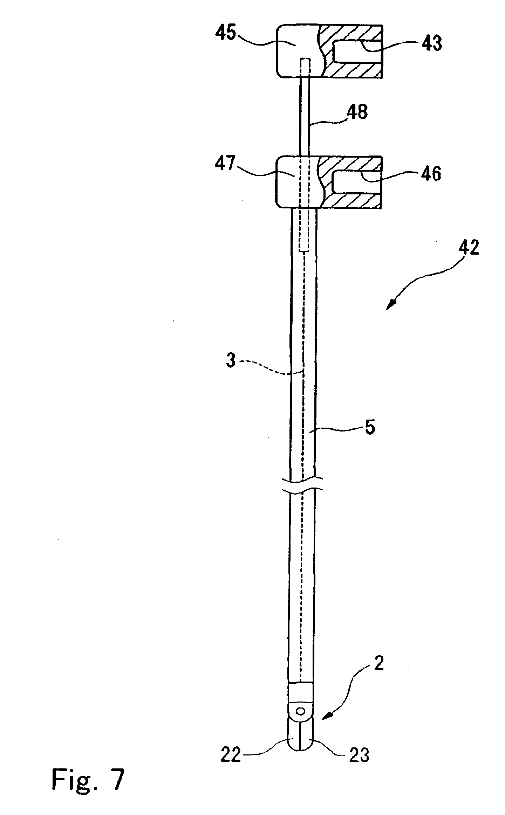

Fig. 7 is a perspective view showing the instrument body of the endoscope treatment system according to the third exemplary embodiment useful for understanding the invention;

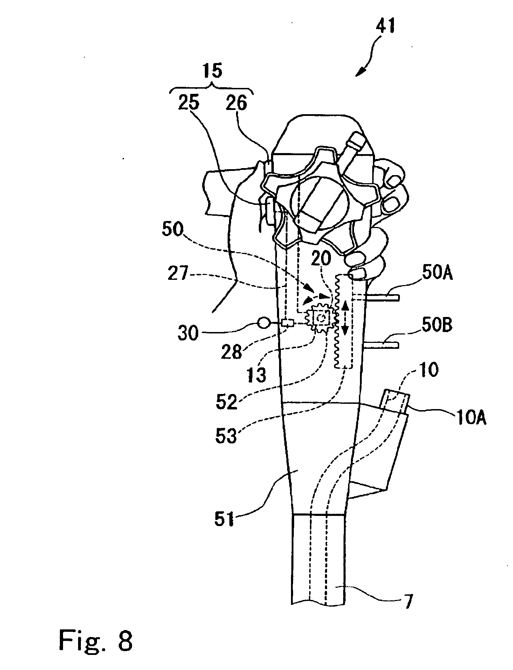

Fig. 8 is a side view showing the endoscope body in the endoscope treatment system according to the third exemplary embodiment useful for understanding the invention;

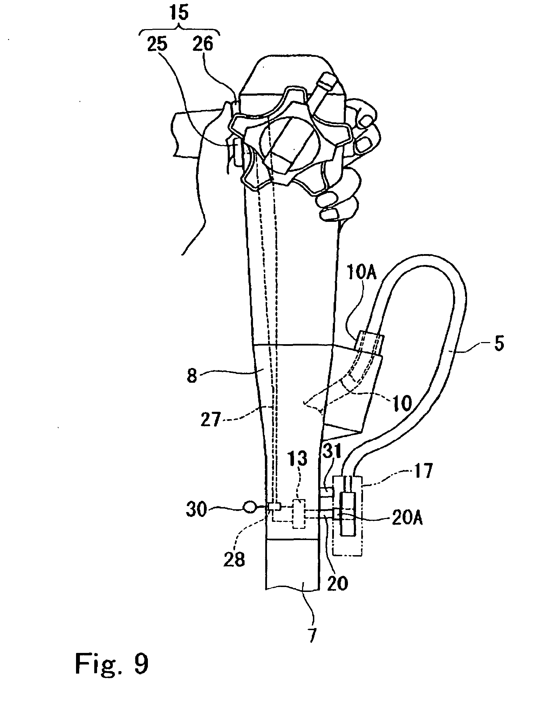

Fig. 9 is a side view partly in cross-section showing the endoscope treatment system according to another exemplary embodiment useful for understanding the invention; and

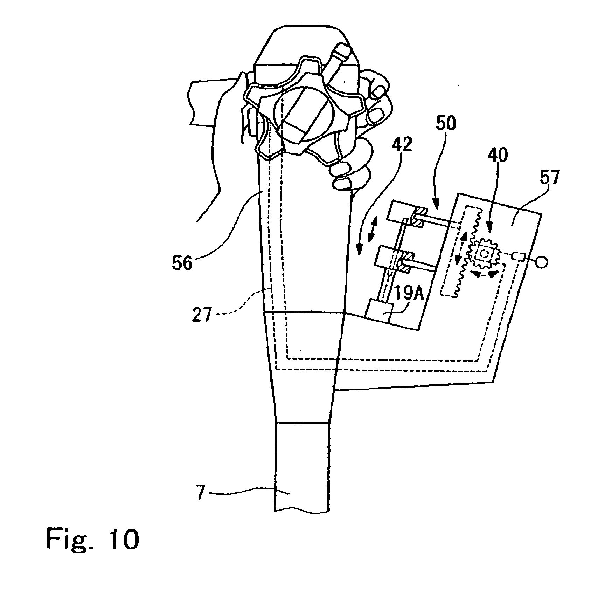

Fig. 10 is a side view partly in cross-section showing the endoscope treatment system according to another exemplary embodiment useful for understanding the invention.

### DESCRIPTION OF THE PREFERRED AND EXEMPLARY EMBODIMENTS

A preferred embodiment of the invention and exemplary embodiments useful for understanding the invention are described below with reference to the accompanying drawings.

Referring to Fig. 1 to Fig. 3, in the embodiment of the present invention, an endoscope treatment system 1 includes an instrument body 6 including a treatment portion 2 which administers a treatment upon receiving a back-and-forth drive force, an operating wire (transmitting member) 3 connected to the treatment portion 2 for transmitting the back-and-forth drive force to the treatment portion 2, and a sheath member 5 having the operating wire 3 fitted therein so that the operating wire can move back-and-forth therein. An endoscope body 11 capable of receiving the instrument body 6 inserted therein has a channel 10 for communicating an insertion portion 7 and an operating portion 8. A back-and-forth mechanism 12 causes the operating wire 3 to move back-and-forth with respect to the sheath member 5 in the axial direction, and a motor 13 provided in the operating portion 8 of the endoscope body 11 drives the back-and-forth mechanism 12. An instruction member 15 issues driving instructions to the motor 13 provided on the operating portion 8.

The instrument body 6 includes a driving member 16 connected to the proximal end of the operating wire 3 for moving the operating wire 3 back-and-forth upon receiving the back-and-forth drive force, a base member 17 which reciprocally accommodates the driving member 16 therein and which is connected to the distal end of the sheath member 5, and a converting unit 18 which converts the rotational force of the motor 13 to the back-and-forth drive force.

The back-and-forth mechanism 12 comprises the driving member 16 and the converting unit 18. The converting unit 18 comprises a rotatable roller 21 engaged with a rotatable shaft 20 of the motor 13. The roller 21 is provided with a square or rectangular hole 21A at the center thereof, to receive a square (or rectangular, etc.) shaft 20A which passes through a hole 17A formed on the base member 17 and which is connected to the revolving shaft 20 of the motor 13.

The square shaft 20A is disposed so as to project outwardly from the portion of the operating portion 8 above a forceps port 10A.

The driving member 16 is formed as a thin plate, and the distal end thereof is connected to the proximal side of the operating wire 3, and the proximal end thereof is connected to the outer periphery of the roller 21. Therefore, the operating wire 3 is connected to the outer periphery of the roller via the driving member 16 and is capable of being wound therearound.

The treatment portion 2 includes a pair of forceps 22, 23, and is connected to the distal end of the operating wire 3. The forceps opens when the operating wire 3 is moved forward toward the distal end with respect to the sheath member 5, and closes when the operating wire 3 is retracted.

The instruction member 15 includes a drive button 25 for activating the motor 13 and a stop button 26 for stopping the motor rotation, and is electrically connected to the motor 13 via wiring 27 disposed within the operating portion 8 of the endoscope body 11.

An electrical power source 28 energizes the motor 13 via the wiring 27 and a changeover switch 30 changes the direction of rotation of the motor 13.

The changeover switch 30 can be switched between a "close" position for rotating the motor 13 in the direction of winding the driving member 16 on the outer periphery of the roller 21 and an "open" position for rotating the motor in the reverse direction.

Disposed above the forceps port 10A of the operating portion 8 is a supporting member 31 for supporting the base member 17 to the operating portion 8 and positioning the square shaft 20A and the square hole 21A so as to be capable of fitting together when operating the operating wire 3.

The method of operation and the effects of the endoscope treatment system 1 according to this embodiment are described below.

Firstly, the operator of the endoscope inserts the insertion portion 7 of the endoscope body 11 into a body cavity of the patient, and then inserts the instrument body 6 from the treatment portion 2 via the forceps port 10A into the channel 10. Subsequently, the square shaft 20A and the square hole 21A are fitted to mount the base member 17 to the supporting member 31.

When performing a treatment with the treatment portion 2, the switch 30 is set to the "open" position, and the drive button 25 of the operating portion 8 is pressed to rotate the motor 13. In response, the motor 13 rotates in a direction which delivers the driving member 16 from the outer peripheral surface of the roller 21. The rotational force of the motor 13 is transmitted to the rotating shaft 20, which then rotates the roller 21 from the shaft 20 through the square shaft 20A and the square hole 21A. The rotational force transmitted to the roller 21 is converted to a force for moving the driving member 16 in the axial direction, and is transmitted to the driving member 16. By unwinding the operating wire 3 off the outer peripheral surface of the roller 21, a forward drive force is transmitted to the treatment portion 2. As a result, the operating wire 3 is moved with respect to the sheath member 5, which opens the forceps 22, 23.

Then, the forceps 22, 23 are moved close to an affected area, at which point the changeover switch 30 is switched to the "close" position. At this time, since the motor 13 rotates in the reverse direction from the direction described above, the roller 21 also rotates in the reverse direction from the direction described above.

Therefore, the driving member 16 is wound on the outer peripheral surface of the roller 21 and the operating wire 3 is retracted with respect to the sheath member 5 to close the forceps 22, 23 to grasp the affected area.

Subsequently, the stop button 26 is pressed to stop the rotation of the motor 13, and engagement between the square shaft 20A and the square hole 21A is released to move the base member 17 apart from the supporting member 31 so that the instrument body 6 can be removed from the channel 10 of the endoscope body 11.

With the endoscope treatment system 1, by operating the instruction member 15 provided on the endoscope body 11, the operator of the endoscope body 11 can perform the operation of the treatment portion 2 by his/her hand holding the operating portion 8 without needing additional assistance. The other hand can be used, for example, for holding the insertion portion 7, whereby the treatment can be performed easily and reliably.

Also, a simple structure is achieved without making the endoscope body 11 structurally complicated.

Referring now to Fig. 4 and Fig. 5, a second exemplary embodiment is described.

The same components as those in the first embodiment described above are represented by the same reference numerals whereby the description thereof is omitted.

The point of distinction of the second exemplary embodiment over the first embodiment is that a converting unit 35 of an endoscope treatment system 32 according to the second exemplary embodiment is provided with a pinion member 36 which can be connected to the square shaft 20A connected to the revolving shaft 20 via the square hole 21A, and a rack member 37, which engages the outer peripheral surface of the pinion member 36, is connected to the proximal end of the operating wire 3 instead of to the driving member 16.

The method of operation and effects of the endoscope treatment system 32 according to this embodiment are now described.

In this exemplary embodiment, as in the first embodiment, an instrument body 33 is inserted into the channel 10, and the base member 17 is supported by the operating portion 8 by the supporting member 31.

When performing the treatment, the changeover switch 30 is set to the "open" position, and the drive button 25 is pressed to rotate the motor 13.

In this case the rotational force of the motor 13 is transmitted to the shaft 20, and from the shaft 20, transmitted via the square shaft 20A to rotate the pinion member 36. The rotational force transmitted to the pinion member 36 is converted to axial movement of the rack member 37 with respect to the base member 17, thereby moving the operating wire 3 in the axial direction to transmit the back-and-forth drive force to the treatment portion 2. In this manner, the operating wire 3 moves forward with respect to the base member 17 to open the forceps 22, 23.

To close the forceps 22, 23, the changeover switch 30 is switched to the "close" position. At this time, since the motor 13 rotates in the reverse direction from the direction described above, the pinion member 36 also rotates in the reverse direction from the direction described above. The rotational force transmitted to the pinion member 36 is converted to an axial force for retracting the rack member 37 with respect to the base member 17 when being transmitted to the rack member 37

engaged therewith, thereby moving the operating wire 3 in the axial direction to transmit the back-and-forth drive force to the treatment portion 2. Consequently, the operating wire 3 is retracted with respect to the base member 17 and the forceps 22, 23.

With the endoscope treatment system 32, the same effects as in the first embodiment are achieved.

Referring now to Fig. 6 to Fig. 8, a third exemplary embodiment is described, in which the same components as those in other preferred and exemplary embodiments described above are represented by the same reference numerals, and the description thereof is therefore omitted.

The difference between the third exemplary embodiment and the first embodiment is that a converting unit 40 of an endoscope treatment system 38 is disposed within an endoscope body 41.

The proximal side of the operating wire 3 of an instrument body 42 is extended so as to project from the sheath member 5. A first engaging member 45, which is formed into a column-shape, is connected and disposed at the proximal end thereof, and is formed on a part of the outer peripheral surface thereof, with a first fitting hole 43. A second engaging member 47, which is formed into a column-shape, is connected and disposed at the proximal end of the sheath member 5, and is also formed on a part of the outer peripheral surface thereof, with a second fitting hole 46.

Disposed between the first engaging member 45 and the second engaging member 47 is a short tube portion 48 which covers the outer periphery of the operating wire 3, and which is capable of moving back-and-forth in the sheath member 5, and the proximal end thereof is connected to the first engaging member 45.

A back-and-forth mechanism 50 includes a first hooking member 50A capable of engaging with the first fitting hole 43, and a second hooking member 50B capable of engaging with the second fitting hole 46 in an operating portion 51 of the endoscope body 41.

The second hooking member 50B supports the sheath member 5 with the operating portion 51, by engaging with and holding the second engaging member 47.

The back-and-forth mechanism 50 includes the rotating shaft 20 connected to the motor 13 and a pinion member 52 connected to the shaft 20. The converting unit 40 is provided with a rack member 53 which is connected to the first hooking member 50A and which can be engaged with the outer peripheral surface of the pinion member 52.

The method of operation and effects of the endoscope treatment system 38 according to this exemplary embodiment will now be described. After the insertion portion 7 of the endoscope body 41 is inserted into the body cavity of a patient, the sheath member 5 of the instrument body 42 is inserted into the channel 10 from the forceps port 10A. Then, the first hooking member 50A and the first fitting hole 43 are fitted to engage the first engaging member 45 with the first hooking member 50A, and the second hooking member 50B and the second fitting hole 46 are fitted to engage the second engaging member 47 with the second hooking member 50B.

When performing a treatment with the treatment portion 2, the motor 13 is rotated in the same manner as in the other preferred and exemplary embodiments described above.

In this case, the rotational force of the motor 13 is transmitted to the shaft 20 to rotate the pinion member 52 connected to the shaft 20. The rotational force transmitted to the pinion member 52 is converted to an axial force which moves the first hooking member 50A back-and-forth in the axial direction, which movement is then transmitted from the first fitting hole 43 to the first engaging member 45.

At this time, since the second engaging member 47 is engaged with the second hooking member 50B via the second fitting hole 46, and hence the sheath member 5 is supported by the operating portion 51, the operating wire 3 is moved with respect to the sheath member 5 axially. In this manner, the operating wire 3 is moved forward with respect to the sheath member 5 to open the pair of the forceps 22, 23.

To close the forceps 22, 23, the changeover switch 30 is switched to the "close" position. At this time, since the motor 13 rotates in the reverse direction from the direction described above, the pinion member 52 rotates in the reverse direction from the direction described above to move the rack member 53 in the reverse direction. Therefore, by retracting the operating wire 3 with the first engaging member 45 with respect to the sheath member 5, the forceps 22, 23 are closed.

Subsequently, the stop button 26 is pressed to stop the rotation of the motor 13. The engagement between the first engaging member 45 and the first hooking member 50A and between the second engaging member 47 and the second hooking member 50B are released, and the instrument body 42 is pulled out from the channel 10 of the endoscope body 41.

With the endoscope treatment system 38, the same effects as those of the first embodiment are achieved. Since the instrument body 42 is not provided with the back-and-forth mechanism 50, the instrument body 42 structure is simpler.

The technical scope of the invention is not limited to the above-described preferred embodiment, and various modifications can be made without departing from the scope of the invention.

For example, in the above-described embodiment, the square shaft 20A is disposed above the forceps port 10A on the operating portion 8 so as to project therefrom, and the motor 13 and the like are also disposed above the forceps port 10A. However, as shown in Fig. 9, a structure such that the motor 13 according to the first embodiment is disposed on the side of the insertion portion 7 with respect to the forceps port 10A, and the supporting member 31 is disposed on the side of the forceps port 10A with respect to the motor 13 is also applicable.

In this case, the direction of projection of the sheath member 5 from the base member 17 is determined to be a direction from the insertion portion 7 to the operating portion 8, the sheath member 5 can be projected from the forceps port 10A by a sufficient length for gripping when moving back-and-forth in the channel 10.

As shown in Fig. 10, a structure in which a supporting portion 57 is provided at a position opposite to an operating portion 56 with the intermediary of the forceps port 19A, and the converting unit 40 and the back-and-forth mechanism 50 according to the third embodiment are disposed on the supporting portion 57 is also applicable.

In this case as well, the instrument body 42 according to the third exemplary embodiment can be used and the endoscope instrument in the related art can be used as well.

In addition, although the electrical power source 28 is disposed in the operating portions 8, 51, it may be disposed in a light source device, not shown, to be connected via a universal cable, not shown with the endoscope bodies 11, 41. In this case, wiring is provided within the universal cable.

While there has been shown and described what is considered to be preferred embodiments of the invention, it will, of course, be understood that various modifications and changes in form or detail could readily be made without departing from the invention as claimed. It is therefore intended that the invention be not limited to the exact forms described and illustrated, but should be constructed to cover all modifications that may fall within the scope of the appended claims.

## Claims

1. An endoscope treatment system comprising:
an instrument body (6) comprising a treatment portion (2) for performing a treatment in response to a back-and-forth drive force, a transmitting member (3) connected to the treatment portion (2) for transmitting the back-and-forth drive force to the treatment portion (2), and a sheath member (5) in which the transmitting member is fitted to move back-and-forth; and
an endoscope body comprising a channel through which the instrument body (6) can be inserted,
wherein the endoscope treatment system further comprises:
a back-and-forth mechanism (12) for causing the transmitting member (3) to move back-and-forth in an axial direction with respect to the sheath member (5);
a driving-power source (13) coupled with the endoscope body for driving the back-and-forth mechanism (12), wherein the driving-power source (13) is a rotational drive source; and
an instruction member (15) coupled with the endoscope body for issuing drive instructions to the power source (13),
**characterized in that** the instrument body (6) comprises:
a driving member (16) connected to the transmitting member (3) and operable to cause the transmitting member (3) to move back-and-forth responsive to the back-and-forth drive force from the back-and-forth mechanism (12);
a base member (17) including the driving member (16) and connected to the sheath member (5); and
a converting mechanism (18) for converting rotational movement of the drive source to the back-and forth drive force,
wherein the driving member (16) is a thin plate and the distal end of the driving member (16) is connected to the proximal side of the transmitting member (3) and the converting mechanism (18) comprises a rotatable roller (21) engaged with a rotatable shaft (20A) connected to the drive source, and wherein a proximal end of the driving member (16) is connected to an outer peripheral surface of the roller (21) so as to be wound therearound.

2. An endoscope treatment system according to Claim 1, wherein the back-and-forth mechanism (12) is disposed in the endoscope body (11).

3. An endoscope treatment system according to Claim 1, wherein the back-and-forth mechanism is disposed in the instrument body (6).

4. An endoscope treatment system according to Claim 1, wherein the endoscope body comprises an operating portion (8) for operating the endoscope, and a forceps port (10A) connected to the proximal end of the channel and disposed on the operating portion (8); and
wherein the back-and-forth mechanism (12) is disposed at a position opposed to the operating portion (8) with the intermediary of the forceps port (10A).

5. An endoscope treatment system according to Claim 1, wherein the back-and-forth mechanism (12) is located on a distal side relative to a forceps opening associated with the endoscope body.

6. An endoscope treatment system according to Claim 1, wherein the instrument body (6) and the endoscope body are structured to be detachably attachable to one another.

## Patentansprüche

1. Endoskopbehandlungssystem umfassend:
einen Instrumentenkörper (6) umfassend einen Behandlungsabschnitt (2) zum Durchführen einer Behandlung in Antwort auf eine Vor-und-zurück-Antriebskraft, ein Übertragungselement (3), das mit dem Behandlungsabschnitt (2) verbunden ist zum Übertragen der Vor-und-zurück-Antriebskraft an den Behandlungsabschnitt (2), und ein Hülsenelement (5), in welchem das Übertragungselement eingebaut ist, um sich vor- und zurückzubewegen; und
einen Endoskopkörper umfassend einen Kanal, durch welchen der Instrumentenkörper (6) eingeführt werden kann,
wobei das Endoskopbehandlungssystem ferner umfasst:
einen Vor-und-zurück-Mechanismus (12) zum Bewirken des Übertragungselements (3), sich in einer axialen Richtung in Bezug auf das Hülsenelement (5) vor-und zurückzubewegen;
eine Antriebsleistungsquelle (13), die mit dem Endoskopkörper gekoppelt ist, zum Antreiben des Vor-und-zurück-Mechanismus (12), wobei die Antriebsleistungsquelle (13) eine Drehantriebsquelle ist; und
ein Instruktionselement (15), das mit dem Endoskopkörper gekoppelt ist, zum Ausgeben von Antriebsinstruktionen an die Leistungsquelle (13),
**dadurch gekennzeichnet, dass** der Instrumentenkörper (6) umfasst:
ein Antriebselement (16), das mit dem Übertragungselement (3) verbunden ist und betätigbar ist, zu bewirken, dass sich das Übertragungselement (3) vor- und zurückbewegt in Antwort auf die Vor-und-zurück-Antriebskraft von dem Vor-und-zurück-Mechanismus (12);
ein Basiselement (17), das das Antriebselement (16) aufweist und mit dem Hülsenelement (5) verbunden ist; und
einen Konvertierungsmechanismus (18) zum Konvertieren einer Drehbewegung der Antriebsquelle in die Vor-und-zurück-Antriebskraft,
wobei das Antriebselement (16) eine dünne Platte ist und das distale Ende des Antriebselements (16) verbunden ist mit der proximalen Seite des Übertragungselements (3) und der Konvertierungsmechanismus (18) eine rotierbare Walze (21) umfasst, die in Eingriff ist mit einem rotierbaren Schaft (20A), der mit der Antriebsquelle verbunden ist, und wobei ein proximales Ende des Antriebselements (16) mit einer äußeren Umfangsoberfläche der Walze (21) verbunden ist, um darum gewunden zu sein.

2. Endoskopbehandlungssystem nach Anspruch 1, wobei der Vor-und-zurück-Mechanismus (12) in dem Endoskopkörper (11) vorgesehen ist.

3. Endoskopbehandlungssystem nach Anspruch 1, wobei der Vor-und-zurück-Mechanismus in dem Instrumentenkörper (6) vorgesehen ist.

4. Endoskopbehandlungssystem nach Anspruch 1, wobei der Endoskopkörper einen Betätigungsabschnitt (8) zum Betätigen des Endoskops umfasst, und einen Zangenanschluss (10A), der mit dem proximalen Ende des Kanals verbunden ist und an dem Betätigungsabschnitt (8) vorgesehen ist; und
wobei der Vor-und-zurück-Mechanismus (12) an einer Position gegenüber dem Betriebsabschnitt (8) mit dem Zwischenstück des Zangenanschlusses (10A) vorgesehen ist.

5. Endoskopbehandlungssystem nach Anspruch 1, wobei der Vor-und-zurück-Mechanismus (12) auf einer distalen Seite relativ zu einer Zangenöffnung positioniert ist, die mit dem Endoskopkörper in Zusammenhang steht.

6. Endoskopbehandlungssystem nach Anspruch 1, wobei der Instrumentenkörper (6) und der Endoskopkörper ausgebildet sind, um lösbar miteinander verbindbar zu sein.

## Revendications

1. Système de traitement endoscopique comprenant :
un corps d'instrument (6) comprenant une partie de traitement (2) destinée à réaliser un traitement en réponse à une force d'entraînement en va-et-vient, un élément de transmission (3) connecté à la partie de traitement (2) destiné à transmettre la force d'entraînement en va-et-vient à la partie de traitement (2), et un élément de gaine (5) dans lequel l'élément de transmission est ajusté pour se déplacer en va-et-vient ; et
un corps d'endoscope comprenant un canal à travers lequel le corps d'instrument (6) peut être inséré,
dans lequel le système de traitement endoscopique comprend en outre :
un mécanisme de va-et-vient (12) destiné à amener l'élément de transmission (3) à se déplacer en va-et-vient dans une direction axiale par rapport à l'élément de gaine (5) ;
une source de puissance d'entraînement (13) couplée avec le corps d'endoscope pour entraîner le mécanisme de va-et-vient (12), dans lequel la source de puissance d'entraînement (13) est une source d'entraînement en rotation ; et
un élément d'instruction (15) couplé au corps d'endoscope pour délivrer des instructions d'entraînement à la source de puissance (13),
**caractérisé en ce que** le corps d'instrument (6) comprend :
un élément d'entraînement (16) connecté à l'élément de transmission (3) et opérable pour amener l'élément de transmission (3) à se déplacer en va-et-vient en réponse à la force d'entraînement en va-et-vient provenant du mécanisme de va-et-vient (12) ;
un élément de base (17) incluant l'élément d'entraînement (16) et connecté à l'élément de gaine (5) ; et
un mécanisme de conversion (18) destiné à convertir un mouvement rotationnel de la source d'entraînement en force d'entraînement en va-et-vient,
dans lequel l'élément d'entraînement (16) est une plaque fine et l'extrémité distale de l'élément d'entraînement (16) est connectée au côté proximal de l'élément de transmission (3) et le mécanisme de conversion (18) comprend un rouleau rotatif (21) engagé avec un arbre rotatif (20A) connecté à la source d'entraînement, et dans lequel une extrémité proximale de l'élément d'entraînement (16) est connectée à une surface périphérique externe du rouleau (21) de façon à être enroulée autour de celui-ci.

2. Système de traitement endoscopique selon la revendication 1, dans lequel le mécanisme de va-et-vient (12) est disposé dans le corps d'endoscope (11).

3. Système de traitement endoscopique selon la revendication 1, dans lequel le mécanisme de va-et-vient est disposé dans le corps d'instrument (6).

4. Système de traitement endoscopique selon la revendication 1, dans lequel le corps d'endoscope comprend une partie d'actionnement (8) destinée à actionner l'endoscope, et un orifice de pince (10A) connecté à l'extrémité proximale du canal et disposé sur la partie d'actionnement (8) ; et
dans lequel le mécanisme de va-et-vient (12) est disposé au niveau d'une position opposée à la partie d'actionnement (8), l'orifice de pince (10A) étant intermédiaire.

5. Système de traitement endoscopique selon la revendication 1, dans lequel le mécanisme de va-et-vient (12) est placé sur un côté distal par rapport à une ouverture de pince associée au corps d'endoscope.

6. Système de traitement endoscopique selon la revendication 1, dans lequel le corps d'instrument (6) et le corps d'endoscope sont structurés pour pouvoir être attachés de manière détachable l'un à l'autre.
